# EUROPEAN PATENT SPECIFICATION

(11) **EP 1 146 038 B1**
(45) Date of publication and mention of the grant of the patent: **16.06.2004**
(21) Application number: 00107869.0
(22) Date of filing: 12.04.2000
(51) Int. Cl.: C07C 323/16, C07C 319/14

(54) **Novel antioxidant and method of producing the same**
Neues Antioxydationsmittel und Verfahren zu dessen Herstellung
Nouvel antioxydant et procédé pour sa préparation

(43) Date of publication of application: 17.10.2001
(73) Proprietor: Kumho Monsanto, Inc., Seoul 100-052 (KR)
(72) Inventor: Shermolovych, Yuriy G., Yochon-shi, Chollanam-do 555-050 (KR); Lim, Sung-Kyu, Seoul 143-210 (KR); Jeon, Jae-Hyuck, Yochon-shi, Chollanam-do 555-040 (KR)
(74) Representative: Merkle, Gebhard

(56) References cited:
- EP-A- 0 224 442
- FR-A- 2 629 817
- GB-A- 911 958
- US-A- 2 322 376
- US-A- 2 417 118
- US-A- 3 553 270
- US-A- 4 759 862
- US-A- 4 874 885
- US-A- 5 008 459
- DATABASE CROSSFIRE [Online] Beilstein; BRN 1888251, 29 June 1989 (1989-06-29) XP002147933

## Description

### BACKGROUND OF THE INVENTION

### 1. Field of the Invention

The present invention relates to an antioxidant and a method of producing the same. More particularly, the present invention relates to an antioxidant having an excellent antioxidant action and an extremely low decolorization, and a method of producing such an antioxidant with a low cost.

### . 2. Description of the Conventional Art

In general, phenols are one of the early substances used in order to delay an oxidation of rubbers. An alkylated phenol is a more effective oxidation preventive agent with less toxicity and a low volatility. A partially hindered alkylated phenol is obtained by a reaction of olefin such as isobutylene, or styrene with phenol, and has 2^{nd} or 3^{rd} alkyl group at the position of ortho or para. The following is representatives of those substances, and formulas II to IV are specified examples of formula I. 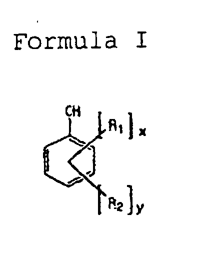 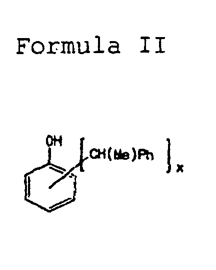 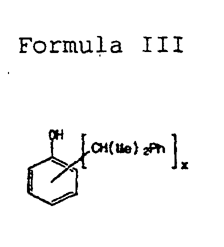 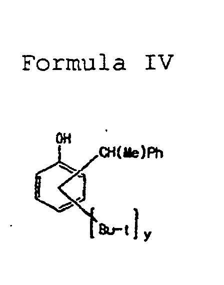 wherein, R1 and R2 are radicals selected from a group consisting of hydrogen and hydro-carbon radicals, and x and y are integers at least 1 or above.

Such compounds have benefits of less decolorization and non-contaminative antioxidant with low cost. However, such compounds have drawbacks in that a large quantity of compounds is required since they act at a weak or medium level as an antioxidant.

In U.S. Patent Nos. 2, 322, 376 and 2,417,118, R. F.Mccleary and S.M.Roberts disclose a compound of the following formula which is a lubricant additive and useful as one having a high antioxidant action. 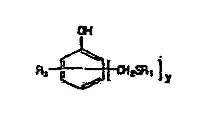 wherein, R is a radical selected from a group consisting of hydrogen and hydrocarbon radicals, R1 is hydrocarbon radical, and x and y are integers of at least 1 or above.

Examples of representatives of the recently disclosed phenolic thioether family compounds are as follows.

U. S. Patent No. 3,553,270

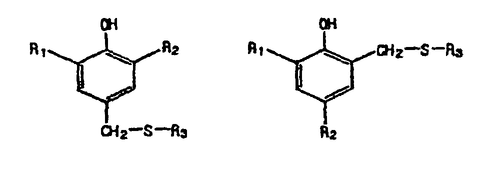

E. P. Patent No. 224,442

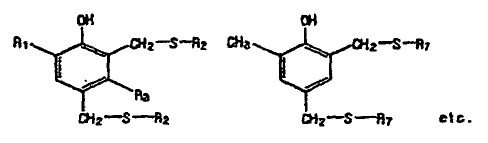

Phenolic thioether which has been disclosed in such material-related patents is composed of a single moiety substance, and has an excellent action for prevention of oxidation. However, such phenolic thioether is too expensive.

Further thioether compounds are also known from US-A-3,553,270 and US-A-4,759,862.

### SUMMARY OF THE INVENTION

Therefore, it is an object of the present invention to provide an antioxidant having an excellent antioxidant action and significantly low decolorization characteristic, and which can be obtained with low cost.

It is another object of the present invention to provide a one-pot method of producing such an antioxidant.

According to the object of the present invention, there is provided an antioxidant which is obtainable as specified in claim 1.

The antioxidant of the present invention has a formula as follows. 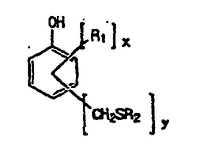 wherein, R₁ is selected from a group consisting of C₃ to C₁₃ alkyl, cycloalkyl, and aralkyl radicals, and R₂ is selected from a group consisting of C₅ to C₁₅ alkyl, cycloalkyl, and aralkyl radicals. Here, x and y are integers of at least 1 or above.

The method of producing an antioxidant according to the present invention includes a process i) of reacting olefins selected from a group consisting of C3 to C13 olefins with phenols selected from a group consisting of phenols of the following formula V. 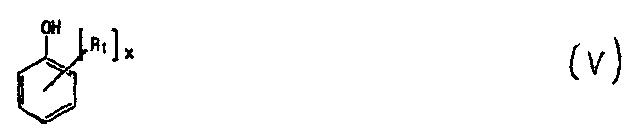 wherein, R₁ and x are as defined as in claim 1.

The method of producing an antioxidant includes a process of reacting the resultant obtained by the process i) with mercaptans selected from a group consisting of mercaptans of the following formula VI and formaldehyde under dialkylamines, more specifically, dipropylamines, preferably at a temperature of 55 °C to 150 °C.

R2 - SH (VI)

wherein, R₂ is selected from a group consisting of C5 to C15 alkyl, cycloalkyl, and aralkyl radicals.

As phenol reactants of the aforementioned formula V, R₁ is isopropyl, n-butyl, sec-butyl, tert-butyl, n-amyl, sec-amyl, 1,1-dimethyl propyl, n-hexyl, 1,1-dimethyl butyl, 1,1-dimethyl pentyl, cyclohexyl, 1,1,3,3-tetramethyl butyl, benzyl, a - methyl benzyl, α , α -dimethyl benzyl, octyl, nonyl, or dodecyl, and x is 0, 1, 2, or 3, and as more preferable phenol reactant, R1 is a mixture of α - methyl styrenated phenols, a mixture of styrenated phenols, a mixture of α - methyl styrenated phenol and styrenated phenol, or a mixture of tert-butylated phenol and styrenated phenol.

Examples of monomercaptans of the above-mentioned formula VI are pentyl mercaptan, hexyl mercaptan, heptyl mercaptan, octyl mercaptan, nonyl mercaptan, dodecyl mercaptan, cyclohexyl mercaptan, 4-methyl cyclohexyl mercaptan, 4-tert-octylcyclohexyl mercaptan, phenyl mercaptan, 4-methyl phenyl mercaptan, 2,4-diethyl phenyl mercaptan, benzyl mercaptan, α - methyl benzyl mercaptan, α ,α - dimethyl benzyl mercaptan, and the like.

The method of producing an antioxidant of the present invention includes a process of mixing an alkylated phenol mixture with formaldehyde and mercaptan under dialkylamine, more specifically, dipropylamine, and heating the resultant composition.

Although the above-described reactions are performed without using a solvent, it is recommended to employ a solvent. It is preferable to use a solvent which has a solvent effect for a reactant and is inactive in practice. More specific examples of such solvents are hydrocarbons like toluene, xylene, octane, dioxane, or tetrahydrofuran. Alternatively, nonprotonic solvent like dimethyl sulfoxide, or C₃ to C₆ primary or secondary alcohol, for example, isopropanol, sec-butyl alcohol, or sec-amyl alcohol, may be employed as solvents.

Mole ratio of reactant is determined to allow an excellent antioxidant action and color characteristics with a low cost.

Mole ratio of phenol : olefin : monomercaptan : formaldehyde is 1:0.5:0.5:0.5 to 1:2.5:2.5:5.0, and total mole amount of olefins and monomercaptan is preferably 1.0 to 5.0 mole per mole of phenol. Total mole amount of olefins and monomercaptan is more preferably 0.5 to 2.5 mole per mole of phenol.

It is required to have a reactivity temperature sufficiently high to allow such reaction to be completed at an appropriate speed, but not causing a decomposition. Preferably, the reactivity temperature is 55° C to 150° C.

A phenolic reaction product according to the present invention preferably is produced according to the following two steps of:
a) performing alkylation to phenols and olefins at a temperature of 85° C to 90° C under an acid catalyst, and
b) after neutralization, reacting phenol/olefin reaction product (alkylated phenol) with monomercaptan and formaldehyde at a termperature of 55° C to 150° C under dialkylamine, more preferably dipropylamine.

Steps a) and b) are completed within 1 to 10 hours after all reactants are added.

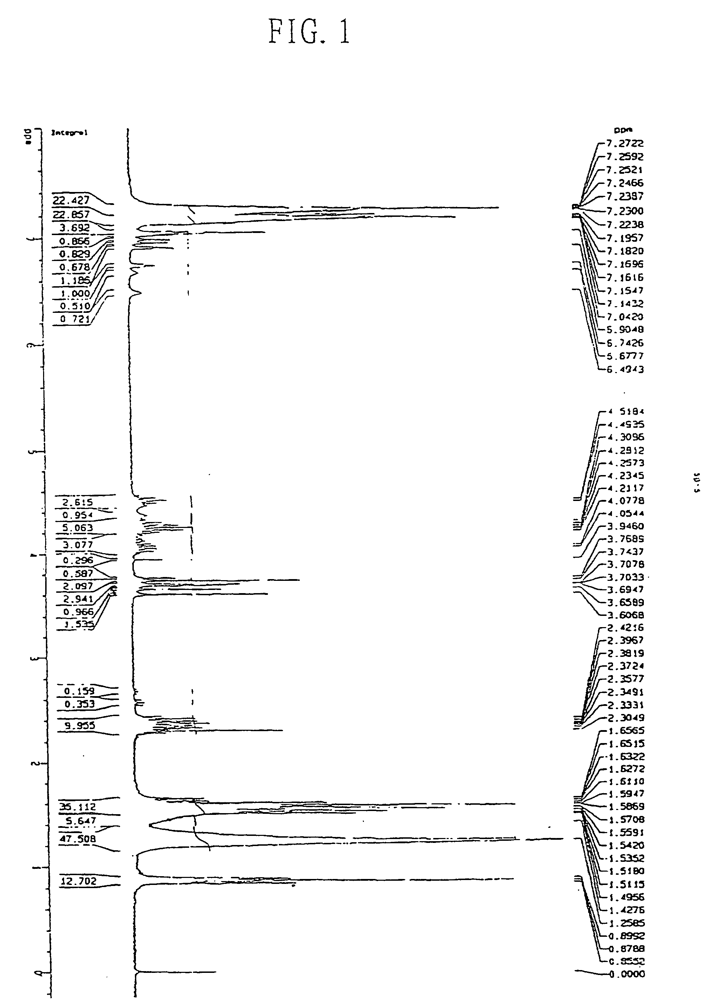

Upon completion of reaction, volatile matters are eliminated from the reaction composition using a mercury of 10mm at a temperature of 90° C, and the resultant is filtered so as to thereby obtain a final product. Fig. 1 illustrates NMR (nuclear magnetic resonance) data of the final product.

### BRIEF DESCRIPTION OF THE DRAWINGS

The present invention will become more fully understood from the detailed description given hereinafter and the accompanying drawings, which are given by way of illustration only, and thus are not limitative of the present invention, and wherein:
Fig. 1 is a graphical representation of NMR data of a novel antioxidant according to the present invention.

### PREFERRED EMBODIMENTS OF THE PRESENT INVENTION

The present invention will be explained in more detail with reference to the attached drawings.

### Embodiment 1

i) phenols 77.3g and sulfuric acid 0.3g are added to a reactor provided with an agitator, cooler, dropping funnel, thermometer, and a facility which can be substituted by nitrogen atmosphere, and the resultant is raised to 85° C to 90° C. Subsequently, styrene 85.3g is added during the time period of 3 hours, being maintained at a temperature of 85° C to 90° C. Then, a post-reaction is performed during the time period of 1.5 hours, being maintained at a temperature of 85° C to 90° C. The resultant is then neutralized by Na2CO3 of 0.19g, and agitated at a temperature of 85° C to 90° C during 1 hour.
ii) The reaction composition obtained by step i) is cooled down to a temperature of 60° C, and 1-octanethiol of 59.7g, and 92% paraformaldehyde of 17.3g are added thereto. The resultant is cooled down to a temperature of 40° C, and dipropylamine (solution) of 16.3g is added thereto. Subsequently, the resultant is cooled down to a temperature of 85° C to 90° C, and a reaction is performed during 4 hours at the same temperature. Upon completion of reaction, volatile matters are eliminated from the reaction composition using a mercury of 10mm at a temperature of 90° C, and the resultant is filtered so as to thereby obtain a final compound. Thus-obtained final compound consists of 2,4,6-tris (α - methyl benzyl) phenol, [2, 4-bis (α - methyl benzyl)-6-octylthiomethyl] phenol, [2,6-bis(α - methyl benzyl)-4-octylthiomethyl] phenol, [2,4-bis(octylthiomethyl)-6-α -methyl benzyl] phenol, [2,6-bis(octylthiomethyl)-4-α -methyl benzyl] phenol, and 2, 4, 6-tris (octylthiomethyl) phenol.

### Embodiment 2

i) phenols 77.3g and sulfuric acid 0.3g are added to a reactor same as that used in embodiment 1, and the resultant is raised to 85° C to 90° C. Subsequently, styrene 113.7g is added during 3 hours or more, being maintained at a temperature of 85° C to 90° C. Then, a post-reaction is performed during 1.5 hours, being maintained at a temperature of 85° C to 90° C.
ii) The reaction composition obtained by step i) is cooled down to a temperature of 60° C, and dipropylamine (solution) of 20g is added thereto (D.P.A neutralization). Subsequently, the resultant is agitated during 10 minutes, and 1-octanethiol of 79.6g and 92% paraformaldehyde of 23.1g are added thereto. The resultant is then raised to 85° C to 90° C, and reaction is performed during 4 hours at the same temperature. Upon completion of reaction, volatile matters are eliminated from the reaction composition using a mercury of 10mm at a temperature of 90° C, and the resultant is filtered so as to thereby obtain a final product same as that of embodiment 1. However, the final product obtained in embodiment 2 has a composition slightly different from that of embodiment 1.

### Embodiment 3

Phenol 77.3 g and sulfuric acid 0.3g, α -methyl styrene 194.2g, 1-octanethiol 119.4g, 92% paraformaldehyde 34.6g, dipropylamine (solution) 16.3g are reacted according to a method same as used in embodiment 1.

Thus-obtained final composition is made up of 2,4,6-tris(α, α - dimethyl benzyl) phenol, [2,4-bis(α , α - dimethyl benzyl)-6-octylthiomethyl] phenol, [2,6-bis(α , α - dimethyl benzyl)-4-octylthiomethyl] phenol, [2,4-bis(octylthiomethyl)-6-α, α-dimethyl benzyl] phenol, [2,6-bis(octylthiomethyl)-4-α ,α -methyl benzyl] phenol, and 2,4,6-tris(octylthiomethyl) phenol.

### Embodiment 4

Phenol 77.3 g and sulfuric acid 0.3g, α -methyl styrene 194.2g, 1-octanethiol 119.4g, 92% paraformaldehyde 34.6g, dipropylamine (solution) 16.3g are reacted according to a method same as used in embodiment 2.

Thus-obtained final composition is the same as that of embodiment 3.

### Embodiment 5

Phenol 77.3 g and sulfuric acid 0.3g, styrene 170.6g, 1-dodecanethiol 165.2g, 92% paraformaldehyde 34.6g, dipropylamine(solution) 20g are reacted according to a method same as used in embodiment 1.

Thus-obtained final composition is made up of 2,4,6-tris(α-methyl benzyl) phenol, [2,4-bis(α, α - methyl benzyl)-6-dodecylthio methyl] phenol, [2,6-bis(α - methyl benzyl)-4-dodecylthio methyl] phenol, [2,4-bis(dodecylthio methyl)-6- α -methyl benzyl] phenol, [2,6-bis(dodecylthio methyl)-4-α -methyl benzyl] phenol, and 2,4,6-tris(dodecylthio methyl) phenol.

### Embodiment 6

Phenol 77.3 g and sulfuric acid 0.3g, α -methyl styrene 194.2g, benzyl mercaptan, 92% paraformaldehyde 34.6g, dipropylamine(solution) 20g are reacted according to a method same as used in embodiment 1, except that, differently from the step ii) of embodiment 1, after the resultant is raised to a temperature of 85° C to 90° C, the post-reaction is carried out during 6 hours.

Thus-obtained final composition is made up of 2,4,6-tris (α , α - dimethyl benzyl) phenol, [2,4-bis (α , α - dimethyl benzyl)-6-benzyl thiomethyl] phenol, [2, 6-bis (α , α - dimethyl benzyl) -4-benzyl thiomethyl] phenol, [2,4-bis(benzyl thiomethyl)-6-α , α-dimethyl benzyl] phenol, [2, 6-bis (benzyl thiomethyl)-4-α ,α -dimethyl benzyl] phenol, and 2,4,6-tris(benzyl thiomethyl) phenol.

Reaction end of the present invention is tested according to the following 2 methods.
1) analysis method 1
a) high-pressure liquid chromatography system (HPLC system) : water alliance system detector : water 996 photodiode array detector
b) eluant : acetonitrile, ethylacetate (gradient)

**< Table 1 >**

| Time (minute) | Acetonitrile(%) | Ethylacetate(%) |
|---|---|---|
| 0 | 100 | 0 |
| 3 | 70 | 30 |
| 10 | 50 | 50 |
| 13 | 80 | 20 |
| 15 | 100 | 0 |
| 20 | 100 | 0 |

c) column : waters spherisorb S50DS2
d) wavelength : 276nm
e) flow rate : 1ml/min
2) analysis method 2
a) high-pressure liquid chromatography system (HPLC system) : water alliance system
b) eluant : MeOH : H20 (90% : 10%) isocratic
c) column : waters spherisorb S50DS2
d) wavelength : 220nm
e) flow rate : 1.2ml/min

Reaction product of the present invention is employed as a stabilizer for oxidative polymer, and 0.005 to 10 parts of the reaction product may be used per 100 parts by weight of polymer.

The following is the result of an aging test performed for SBR-1502 rubber.
1. Comparison between a composition of Octylthiomethylated, styrenated phenols and a composition of styrenated phenols.
The aging test is carried out at a temperature of 100° C with respect to SBR-1502 rubber.

**< Table 2 >**

| change in Mooney viscosity | | | | | |
|---|---|---|---|---|---|
| hour | S1 | S2 | S3 | S4 | S5 |
| 0 | 55.2 | 55.3 | 56.1 | 54.8 | 52.2 |
| 50 | 72.5 | 59.6 | 63.0 | 47.0 | 47.7 |
| 96 | 76.3 | 65.7 | 72.5 | 43.9 | 39.6 |
| 168 | 78.1 | 77.9 | 80.7 | 66.1 | 40.2 |

**< Table 3 >**

| Explanation on test samples S1 to S5 | | | |
|---|---|---|---|
| Test samples | Reactant | Mole ratio | Quantity(PHR) |
| S1 | Phenol:styrene | 1:2 | 1.25 |
| S2, S3 | Phenol:styrene: | 1:2:0.08:0.04 | S2 : 1.25 |
| | formaldehyde:1-octanethiol | | S3 : 0.50 |
| S4, S5 | Phenol:styrene: | 1:2:1.3:1 | S4 : 0.50 |
| | formaldehyde:1-octanethiol | | S5 : 1.25 |

When a variety of aging tests are performed on SBR rubber using a reaction product of the present invention, i.e., a composition of octylthiomethylated, styrenated phenols and a composition of styrenated phenols, test samples S1 to S5 resulted in a similarity with respect to physical characteristics (say, tensile strength, extension(%), hardness, and module, and the like) of the rubber. However, test sample S4 shows a significant enhancement in color and Mooney viscosity, as compared with test samples S5 and S1.
2. Comparison between a composition of octylthiomethylated, styrenated phenols and a composition of 4,6-bis(octylthiomethyl)-o-cresol.
The aging test is carried out at a temperature of 70° C during 12, 24, 48, and 72 hours, and at a temperature of 100° C during 6, 12, 24, and 48 hours with respect to SBR-1502 rubber.

**< Table 4 >**

| Test samples S1' to S3' | | | |
|---|---|---|---|
| Test samples | S1' | S2' | S3' |
| quantity | a: 0.6 PHR | a : 0.15 PHR + | b : 0.15 PHR + |
| | | c : 0.45 PHR | c : 0.45 PHR |

wherein, a denotes composition of octylthiomethylated, styrenated phoenols (mole ratio of the employed reactant is the same as those of S1 and S5), b denotes 4,6-bis(octylthiomethyl)-o-cresol, and c denotes tris(nonylated phenyl) phosphite.
When a variety of aging tests are performed on SBR-1502 rubber using a reaction product of the present invention, i.e., a composition of octylthiomethylated, styrenated phenols and a composition of 4,6-bis(octylthiomethyl)-o-cresol, test samples S1' to S3' resulted in a similarity with respect to physical characteristics (say, tensile strength, extension(%), hardness, and module, and the like) of the rubber, and color and Mooney viscosity.
As described above, the present invention is advantageous in that a composition having an excellent antioxidant action and least decolorization can be obtained with low cost.

## Claims

1. A compound of the following formula: 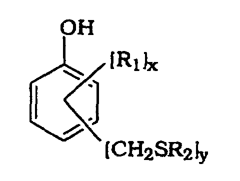 wherein R₁ is selected from the group consisting of C₃ to C₁₃ alkyl, cycloalkyl. and aralkyl radicals, R₂ is selected from the group consisting of C₅ to C₁₅ alkyl, cycloalkyl, and aralkyl radicals, and x and y are integers of at least 1 or above.
obtainable by a method comprising the steps of:
a) reacting olefins selected from the group consisting of C₃ to C₁₃ olefins with phenols selected from the group consisting of phenols of the following formula (V) 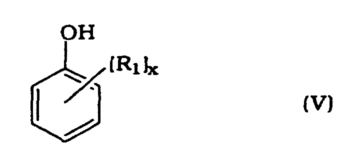 wherein R₁ is as defined above and x is 0, 1, 2 or 3, in the presence of an acid catalyst, in order to obtain a composition of C₉ to C₄₅ alkylated phenols: and
b) reacting the composition obtained in step a) with monomercaptans of the following formula (VI)
R₂-SH (VI)
wherein R₂ is as defined above, and formaldehyde in the presence of a dialkylamine catalyst, wherein the mole ratio of phenol:olefin:monomercaptan:formaldehyde is in the range of from 1:0.5:0.5:0.5 to 1:2.5:2.5:5.0.

2. The compound according to claim 1, wherein said R₁ is aralkyl.

3. The compound according to claim 1, wherein said R₁ is α-methylbenzyl.

4. The compound according to claim 1, wherein said R₁ is α,α-dimethylbenzyl.

5. A method of manufacturing the compound of claim 1, said method comprising the steps of:
a) reacting olefins selected from the group consisting of C₃ to C₁₃ olefins with phenols selected from the group consisting of phenols of the following formula (V) 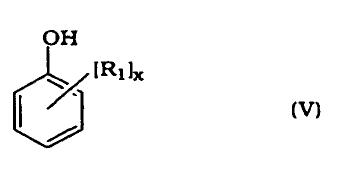 wherein R₁ is as defined in claim 1 and x is 1. 2 or 3. in the presence of an acid catalyst, in order to obtain a composition of C₉ to C₄₅ alkylated phenols; and
b) reacting the composition obtained in step a) with monomercaptans of the following formula (VI)
R₂-SH (VI)
wherein R₂ is as defined in claim 1. and formaldehyde in the presence of a dialkylamine catalyst, wherein the mole ratio of phenol:olefin:monomercaptan:formaldehyde is in the range of from 1:0.5:0.5:0.5 to 1:2.5:2.5:5.0.

6. The method according to claim 5, wherein said olefin is α-methylstyrene.

7. The method according to claim 5 or 6, wherein the total mole quantity of said olefin and monomercaptan is 1.0 to 5.0 per mole of phenol.

8. The method according to any of claims 5-7, wherein the reaction temperature in step b) is 55°C to 150°C.

9. The method according to any of claims 5-8, wherein the dialkylamine catalyst in step b) is dipropylamine.

10. An antioxidant composed of at least one or two compounds from among the compounds of claim 1.

## Patentansprüche

1. Verbindung der folgenden Formel: 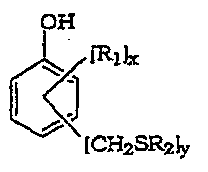 worin R₁ aus der Gruppe gewählt ist, bestehend aus C₃-C₁₃-Alkyl-, Cycloalkylund Aralkylresten, R₂ aus der Gruppe gewählt ist, bestehend aus C₅-C₁₅-Alkyl-, Cycloalkyl- und Aralkylresten, und x und y ganze Zahlen von mindestens 1 oder darüber sind.
erhältlich durch ein Verfahren, umfassend die Schritte:
a) Umsetzen von Olefinen, gewählt aus der Gruppe, bestehend aus C₃-C₁₃-Olefinen, mit Phenolen, gewählt aus der Gruppe, bestehend aus Phenolen der folgenden Formel (V) 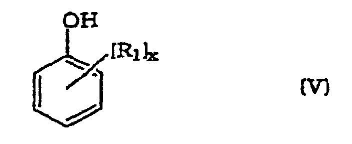 worin R₁ wie oben definiert ist, und x 0, 1, 2 oder 3 ist, in Gegenwart eines Säurekatalysators, um eine Zusammensetzung aus C₉-C₄₅-alkylierten Phenolen zu erhalten; und
b) Umsetzen der in Schritt a) erhaltenen Zusammensetzung mit Monomercaptanen der folgenden Formel (VI)
R₂-SH (VI)
worin R₂ wie oben definiert ist. und Formaldehyd in Gegenwart eines Dialkylaminkatalysators, wobei das Molverhältnis von Phenol:Olefin:Monomercaptan:Formaldehyd im Bereich von 1:0,5:0,5:0,5 bis 1:2,5:2,5:5,0 liegt.

2. Verbindung nach Anspruch 1, wobei R₁ Aralkyl ist.

3. Verbindung nach Anspruch 1, wobei R₁ α-Methylbenzyl ist.

4. Verbindung nach Anspruch 1, wobei R₁ α,α-Dimethylbenzyl ist.

5. Verfahren zum Herstellen der Verbindung nach Anspruch 1, wobei das Verfahren die Schritte umfasst:
a) Umsetzen von Olefinen, gewählt aus der Gruppe, bestehend aus C₃-C₁₃-Olefinen, mit Phenolen, gewählt aus der Gruppe, bestehend aus Phenolen der folgenden Formel (V) 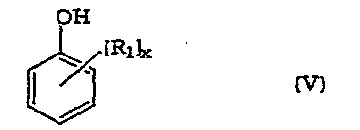 worin R₁ wie in Anspruch 1 definiert ist, und x 1, 2 oder 3 ist, in Gegenwart eines Säurekatalysators, um eine Zusammensetzung aus C₉-C₄₅-alkylierten Phenolen zu erhalten; und
b) Umsetzen der in Schritt a) erhaltenen Zusammensetzung mit Monomercaptanen der folgenden Formel (VI)
R₂-SH (VI)
worin R₂ wie in Anspruch 1 definiert ist, und Formaldehyd in Gegenwart eines Dialkylaminkatalysators, wobei das Molverhältnis von Phenol:Olefin:Monomercaptan:Formaldehyd im Bereich von 1:0,5:0,5:0,5 bis 1:2,5:2,5:5,0 liegt.

6. Verfahren nach Anspruch 5, wobei das Olefin α-Methylstyrol ist.

7. Verfahren nach Anspruch 5 oder 6, wobei die gesamte Molmenge aus dem Olefin und Monomercaptan 1.0 bis 5.0 pro Mol Phenol beträgt.

8. Verfahren nach mindestens einem der Ansprüche 5-7, wobei die Reaktionstemperatur in Schritt b) 55°C bis 150°C beträgt.

9. Verfahren nach mindestens einem der Ansprüche 5-8, wobei der Dialkylaminkatalysator in Schritt b) Dipropylamin ist.

10. Antioxidationsmittel, zusammengesetzt aus mindestens einer oder zwei Verbindungen aus den Verbindungen nach Anspruch 1.

## Revendications

1. Un composé ayant la formule suivante : 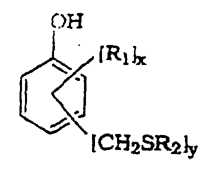 dans laquelle R₁ est choisi dans le groupe constitué par des radicaux alkyle, cycloalkyle, et aralkyle, en C₃ à C₁₃, R₂ est choisi dans le groupe constitué par des radicaux alkyle, cycloalkyle et aralkyle en C₅ à C₁₅, et x et y sont des nombres entiers au moins égaux à 1 ou plus, pouvant être obtenus par une méthode comprenant les étapes suivantes :
a) réaction d'oléfines choisies dans le groupe constitué par les oléfines en C₃ à C₁₃ avec des phénols choisis dans le groupe constitué par les phénols ayant la formule (V) suivante : 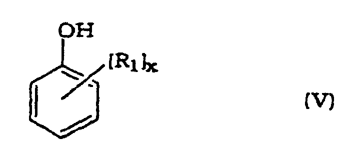 dans laquelle R₁ est comme défini ci-dessus et x est égal à 0, 1, 2, ou 3 en présence d'un catalyseur acide, pour obtenir une composition de phénols alkyiés en C₉ à C₄₅ ; et
b) réaction de la composition obtenue dans l'étape a) avec des monomercaptans de la formule (VI) suivante :
R₂-SH (VI)
dans laquelle R₂ est comme défini ci-dessus avec de la formaldéhyde en présence de dialkylamine en tant que catalyseur, dans laquelle le rapport molaire de phénols:oléfines:monomercaptans:formaldéhyde est dans l'intervalle de 1:0,5:0,5:0,5 à 1:2,5:2,5:5,0.

2. Le composé selon la revendication 1, dans lequel ledit R₁ est un aralkyle.

3. Le composé selon la revendication 1, dans lequel ledit R₁ est un α-méthylbenzyle.

4. Le composé selon la revendication 1, dans lequel ledit R₁ est α,α-diméthylbenzyle.

5. Une méthode de fabrication du composé de la revendication 1, ladite méthode comprenant les étapes suivantes :
a) réaction d'oléfines choisies dans le groupe constitué par des oléfines en C₃ à C₁₃ avec des phénols choisis dans le groupe constitué par des phénols de la formule (V) suivante : 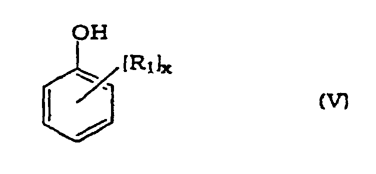 dans laquelle R₁ est comme défini dans la revendication 1 et x est égal à 1, 2 ou 3, en présence d'un catalyseur acide pour obtenir une composition de phénols alkylés en C₉ à C₄₅ ; et
b) réaction du composé obtenu dans l'étape a) avec des monomercaptans ayant la formule (VI) suivante :
R₂-SH (VI)
dans laquelle R₂ est comme défini dans la revendication 1 et du formaldéhyde en présence de dialkylamine en tant que catalyseur dans laquelle le rapport molaire de phénols:oléfines:monomercaptans:formaldéhyde est dans l'intervalle de 1:0,5:0,5:0,5 à 1:2,5:2,5:5,0.

6. La méthode selon la revendication 5 dans laquelle ladite oléfine est de l'α-méthylstyrène.

7. La méthode selon la revendication 5 ou 6, dans laquelle la quantité molaire totale de ladite oléfine et ledit monomercaptan est de 1,0 à 5,0 par mole de phénols.

8. La méthode selon l'une quelconque des revendications 5 à 7, dans laquelle la température de la réaction dans l'étape b) est de 55° C à 150° C.

9. La méthode selon l'une quelconque des revendications 5 à 8, dans laquelle ladite dialkylamine en tant que catalyseur dans l'étape b) est la dipropylamine.

10. Un anti-oxydant composé d'au moins un ou deux composés choisis parmi les composés de la revendication 1.
